# FASCICULE DE BREVET EUROPEEN

(11) **EP 2 819 654 B1**
(45) Date de publication et mention de la délivrance du brevet: **09.09.2015**
(21) Numéro de dépôt: 13715262.5
(22) Date de dépôt: 25.02.2013
(51) Int. Cl.: A61K 9/16, A61K 9/20, A61K 31/165

(54) **PROCÉDÉ D'OBTENTION D'UNE COMPOSITION PHARMACEUTIQUE À BASE DE MODAFINIL, COMPOSITION PHARMACEUTIQUE AINSI OBTENUE ET SON APPLICATION**
HERSTELLUNGSVERFAHREN EINER PHARMAZEUTISCHEN ZUBEREITUNG ENTHALTEND MODAFINIL, DIE RESULTIERENDE ZUBEREITUNG UND IHRE VERWENDUNG
METHOD FOR THE PREPARATION OF A PHARMACEUTICAL COMPOSITION COMPRISING MODAFINIL, COMPOSITION OBTAINED BY THIS METHOD AND ITS APPLICATION

(30) Priorité: 28.02.2012 FR 1200580
(43) Date de publication de la demande: 07.01.2015
(73) Titulaire: DEBREGEAS ET ASSOCIES PHARMA, 75008 Paris (FR)
(72) Inventeur: LEBON, Christophe, F-28260 Rouvres (FR); SUPLIE, Pascal, F-27400 Montaure (FR); LEBOEUF, Fabrice, F-54130 Saint-Max (FR); JUNG, Jennifer, F-54380 Dieulouard (FR); DESCHAMPS, Frantz, F-54000 Nancy (FR)
(74) Mandataire: Gallochat, Alain
(86) Numéro de dépôt international: PCT/FR2013/000051
(87) Numéro de publication internationale: WO 2013/128087

(56) Documents cités:
- WO-A2-2007/013962
- US-A- 5 618 845
- US-A1- 2006 024 370
- US-A1- 2007 275 057

## Description

Le modafinil est le 2-[(diphénylméthyl)sulfinyl)]acétamide, la formule moléculaire est C15H15NO2S et sa formule développée :

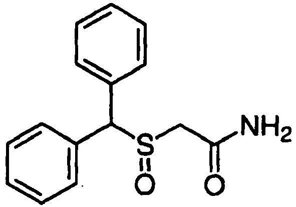

En 1970 les dérivés benzhydryl sulfinyl ont été développés en France par les laboratoires pharmaceutiques Lafon. En 1986, l'Adrafinil a été testé dans le traitement de la narcolepsie et abandonné en raison de problèmes d'ordre gastro-intestinal, de phénomène de rash et d'importantes allergies cutanées. Le modafinil est un médicament éveillant utilisé en Europe depuis 1992 ; il augmente les niveaux d'éveil et de vigilance diurne et ainsi est actuellement prescrit dans le traitement de la narcolepsie. Le mode d'action n'est pas totalement expliqué mais fait intervenir une inhibition de la recapture de la noradrénaline au niveau des noyaux inducteurs de sommeil du noyau ventrolatéral optique ; il présenterait également un effet agoniste alpha 1 adrénergique, un effet positif sur la transmission glutamatergique et il se lie au transporteur de la dopamine et en diminue la recapture.

Il est commercialisé sous les noms de Modiodal, Provigil et Alertec. La dose administrée varie d'une prise de 100 mg à deux prises de 200 mg par jour ; la demi-vie d'élimination est d'environ 14 heures chez l'homme.

Le modafinil est distribué sous sa forme racémique qui présente un centre chiral qui est en fait un atome de soufre ; il existe cependant deux isomères optiquement actifs : l'énantiomère dextrogyre et la forme lévogyre, ces deux formes étant a priori présentes en quantité égale dans le racémique.

Les deux énantiomères ont la même activité pharmacologique chez l'animal. Cependant chez l'homme, l'énantiomère lévogyre (R) présente une demi-vie de 10 à 14 heures ; l'énantiomère dextrogyre (S) présente quant à lui une demi-vie de 3 à 4 heures [réf biblio : Wong et al., J.Clin. pharmacol.,39:30-40(1999) ; Wong et al., J.Clin. pharmacol.,39 : 281-288(1999) ; Robertson et al., Clin Pharmacokinet.,42 : 123-137 (2003)].

Après administration, l'énantiomère R présenterait une AUC supérieure au racémique et une moindre variabilité des taux plasmatiques.

Le modafinil est utilisé dans le traitement de la somnolence diurne excessive associée à une narcolepsie avec ou sans catalepsie. La somnolence diurne excessive se caractérise par une difficulté à rester éveillé et une augmentation des endormissements survenant à des moments inopportuns. La dose initiale recommandée est de 200 mg par jour administrée en une seule prise le matin ou en deux prises matin et midi selon la réponse du patient. Les doses peuvent être augmentées jusqu'à 600 mg pour les patients présentant une réponse insuffisante.

Le problème des formes commerciales disponibles actuellement réside dans la persistance de l'effet du modafinil bien au-delà de la période souhaitée par le patient et même cette rémanence de la vigilance est finalement de nature à perturber le cycle normal de sommeil du patient et même peut induire une insomnie.

Le modafinil a été également utilisé avec succès chez l'enfant dans le traitement du ADHD (attention déficit hyperactivity disorder) ; dans toutes ces pathologies on observe au niveau des signes cliniques une forte variabilité et donc il s'avère nécessaire de procéder à un ajustement thérapeutique individuel.

Un objet de la présente invention est de mettre à la disposition du patient une nouvelle forme médicamenteuse orale de S modafinil possédant une biodisponibilité augmentée comparativement au racémique et une durée d'action raccourcie.

L'un des objectifs de la présente invention est également de fournir une formulation capable de répondre à la forte variabilité inter individuelle et donc de mettre à disposition une formulation homothétique permettant un ajustement aisé de la dose administrée.

Un autre objet de l'invention est de mettre à la disposition du patient une préparation thérapeutique permettant un effet thérapeutique très rapide comparativement au racémique et comparativement à l'énantiomère S administré seul.

Ainsi la présente invention concerne des formes thérapeutiques orales de S Modafinil se présentant sous la forme de comprimés, sachets ou bien sous la forme de gélules dosées entre 25 et 200 mg de principe actif, le modafinil S, et préférentiellement de 50 à 100 mg.

Les formulations ici présentées sont homothétiques et donc sont identiques quels que soient les dosages administrés au patient, ce qui concourt à diminuer la forte variabilité signalée précédemment.

Les compositions qui vont être décrites ont été spécifiquement développées de manière à obtenir *in vitro* une dissolution très rapide et dans tous les cas supérieure à celle obtenue avec une forme commercialisée sur le marché ; une méthode de dissolution discriminante a donc été spécifiquement développée et a permis de sélectionner les excipients et procédés de fabrication.

De nombreuses formulations orales de modafinil sont décrites dans l'art antérieur ; ainsi le brevet US 7297 346 décrit une composition de modafinil, comprenant du lactose, de l'amidon de maïs, du silicate de magnésium, de la croscarmellose de sodium, du pvp, du talc et du stéarate de magnésium.

Cette composition utilise le modafinil sous sa forme racémique et correspond à la formulation commerciale Provigil.

Le texte US N° RE37516 présente des compositions pharmaceutiques utilisant des particules de granulométrie bien définie, et particulièrement des compositions pour lesquelles 95 % des particules présentent une taille inférieure à 200 microns. Dans ce document l'effet pharmacologique obtenu est directement lié à et dépendant de la taille des particules finales du principe actif; il est ainsi décrit que les particules de granulométrie définie sont obtenues par de multiples opérations successives de broyage et tamisage. Ces opérations sont lourdes et fastidieuses et difficilement compatibles économiquement avec la réalité d'une production industrielle. Le procédé de la formulation faisant l'objet de la présente invention utilise une toute autre technique, dite du fluide supercritique qui sera décrite plus avant.

La famille des brevets US 6919378, US6489363 et EP1562572B1 vise une administration sous forme de solutions préférentiellement non aqueuses, dont la portée est très large : solutions se présentant sous forme de mélange de deux ou plusieurs substances, lesdites solutions pouvant être sous forme d'un solide dispersé dans un liquide, un solide ou un milieu semi-solide. Ainsi le brevet EP 1562572 mentionne une dispersion solide comprenant au moins un support solide, ce même support appartenant à la famille des PEG de poids moléculaire compris entre 3000 et 8000 daltons, le PEG 400 ayant été exclu.

Le brevet US 6919378 décrit des compositions non aqueuses. Le brevet US 6489363 décrit quant à lui une composition pharmaceutique comprenant du modafinil en solution préférentiellement non aqueuse mais les compositions aqueuses sont également revendiquées.

La demande de brevet US 2007/0275057 décrit une composition orale comprenant un ou plusieurs excipients pharmaceutiques dans laquelle au moins 65 % des particules de modafinil présentent un diamètre supérieur à 220 microns. Dans la composition faisant l'objet de la présente invention, le modafinil S est solubilisé dans un fluide super critique puis cristallisé sur un support après détente du fluide. Dans ce procédé les particules de modafinil S obtenues sont de l'ordre du micron.

Le brevet EP 1542666 décrit une composition pharmaceutique comprenant au moins deux populations de particules de modafinil de plages de dimensions bornées, certaines de ces plages étant exclues de la composition pharmaceutique finale. Cela suppose, en termes de fabrication, plusieurs étapes de séparation et de contrôles de granulométrie et implique un procédé long et difficile.

La demande de brevet US 2004/0121003 décrit une méthode particulière pour l'obtention d'un mélange d'un principe actif et d'au moins un excipient de taille moyenne comprise entre 10 et 500 microns ; ce même mélange étant broyé (broyage à jet d'air) pour maintenir la taille et l'aspect des particules individuelles.

La demande de brevet internationale WO 2008/008879 concerne des compositions de nanoparticules comprenant le modafinil ou un de ses sels et au moins un agent stabilisateur de surface absorbé sur ces mêmes nanoparticules ; la granulométrie moyenne des particules est au moins de 2000 nm. Dans les compositions de la présente invention, le principe actif est solubilisé dans le fluide supercritique puis est absorbé sur un support.

La présente invention va maintenant être mieux comprise à l'aide des éléments qui vont suivre.

Le modafinil se présente sous forme d'une poudre blanche cristalline pratiquement insoluble dans l'eau et partiellement soluble dans le méthanol et l'acétone. Il en résulte une faible biodisponibilité du modafinil ; on l'estime à environ 40% ; en effet la solubilité du modafinil étant trop faible, la biodisponibilité absolue n'a pas pu être déterminée.

On a ainsi cherché à améliorer la solubilité du modafinil ; en particulier, et c'est l'un des objets de l'invention, la solubilité du modafinil a été étudiée dans les fluides supercritiques.

Parmi ces fluides supercritiques, on peut citer le CO₂ : la technologie du CO₂ supercritique est basée sur le pouvoir solvant du CO₂ qui est modulable à volonté selon les conditions de pression et de température qu'on lui applique.

A l'état supercritique (plus de 74 bars et de 31°C) le CO₂ possède des propriétés très particulières. Le fluide obtenu est caractérisé par une grande diffusivité (de l'ordre de celle des gaz), ce qui lui confère une bonne aptitude à la diffusion, et une densité élevée qui le dote d'une capacité de transport et d'extraction importante.

Un procédé d'extraction par CO₂ supercritique fonctionne en circuit fermé. Il comporte des organes de mise en pression (pompes) et en température (échangeurs) afin d'amener le CO₂ au-dessus de son point critique.

Le produit à traiter est placé dans un extracteur traversé par le flux de CO₂ supercritique. Les molécules solubles dans le CO₂ supercritique, donc extractibles, sont les composés peu polaires de faible masse moléculaire.

Le fluide supercritique possède plusieurs avantages par rapport au fluide liquide :
- un grand coefficient de diffusivité et un petit coefficient de viscosité ;
- une absence de tension de surface, ce qui augmente le pouvoir de pénétration du fluide supercritique.

Un fluide supercritique présente un autre avantage par rapport aux autres solvants : sa solubilité change selon que l'on fait varier sa température ou sa pression. On peut ainsi faire en sorte qu'il soit un solvant pour certaines substances à un moment donné, et plus du tout l'instant d'après. Cela facilite la récupération de la substance qui a été dissoute.

On s'est ainsi rendu compte que le modafinil présente une solubilité acceptable dans le CO₂ comme le montre la figure ci-dessous.

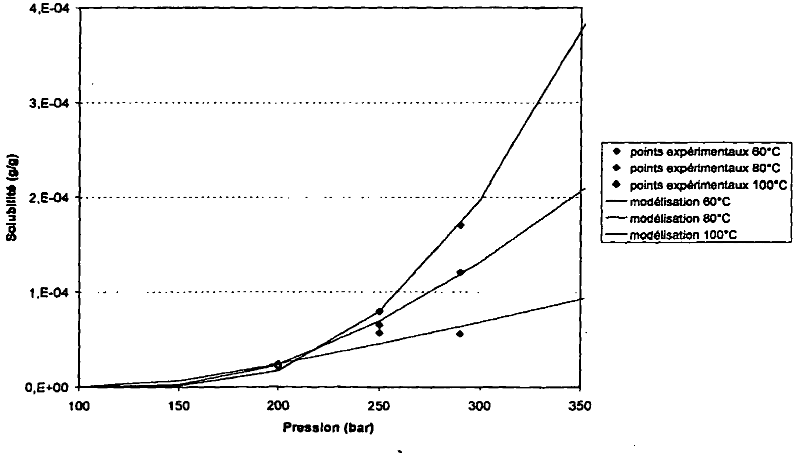

On a ensuite cherché à pulvériser le principe actif dissous sur un support inerte de manière à avoir des granules constituées dudit support à la surface desquelles étaient absorbées des particules de modafinil obtenues selon le procédé précité. La granulométrie de ces particules ainsi que la forme cristalline de ces mêmes particules a fait l'objet d'une étude approfondie.
Plusieurs essais ont été réalisés, d'abord en utilisant du lactose anhydre comme support.

Différents échantillons ont été réalisés en faisant varier les paramètres : température et pression d'extraction, température de pré-expansion, pression d'expansion, pourcentage de CO₂ liquide.

En étudiant les formes cristallines des poudres obtenues par l'analyse thermique différentielle ou par diffraction aux rayons X et en comparant ces mêmes données à celles de la littérature, on peut donc conclure que les profils sont identiques et correspondent au lactose anhydre et à la forme IV du modafinil racémique.

La dissolution a ensuite été étudiée en HPLC, paniers 50rpm ; Milieu de dissolution : HCl 0,1N ; Température du bain : 37°C ; volume : 450 ml ; Prise d'essai équivalente à environ 50 mg de modafinil S ; Formulations mises en gélules taille 000 (deux gélules).

Les résultats obtenus apparaissent sur la figure suivante : 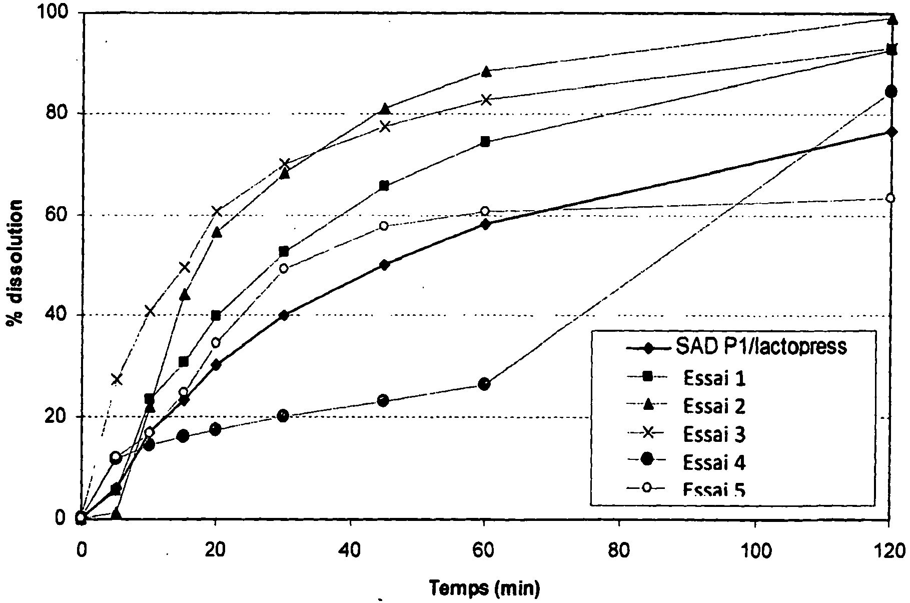 Une augmentation *in vitro* de la dissolution comparativement au mélange seul (SAD P1/lactopress) est observée.
Différents paramètres ont ensuite été modifiés :
- changement du support pour du mannitol,
- augmentation du taux de charge jusque 30% de charge de principe actif
- utilisation du S modafinil
- solvant autre que le CO₂, notamment le tétrafluoroéthane, plus spécifiquement le 1,1,1,2-tétrafluoroéthane.
Le tableau ci-dessous reprend les résultats en utilisant du S modafinil, et du mannitol à la place du lactose anhydre ; en outre le taux de charge a été augmenté aux environs de 30 %.

| Essai | Quantité de modafinil extrait (g) | Solubilité (g/g) | Quantité de formulation collectée (g) | Rendement de collecte (%) | Taux de charge théorique (%) |
|---|---|---|---|---|---|
| | Formulations préparées avec CO₂ supercritique | | | | |
| Formulation 1 | 10,0 | 1.0.E-04 | 29,54 | 96 | 32,65 |
| Formulation 2 | 9,5 | 9,3.E-05 | 29,40 | 98 | 31,52 |
| Formulation 3 | 9,7 | 9,9.E-05 | 29,62 | 98 | 32,18 |
| | Formulations préparées avec le tétrafluoroéthane | | | | |
| Formulation 4 | 11,4 | 2,5.E-04 | 28,81 | 86 | 31,38 |
| Formulation 5 | 11,5 | 1,9.E-04 | 26,68 | 80 | 31,66 |
| Formulation 6 | 11,5 | 2,7.E-04 | 30,36 | 91 | 34,27 |

Les courbes ci-dessous illustrent la comparaison des profils DSC des formulations de type S-modafinil/mannitol préparées par les procédés CO₂ supercritique ou tétrafluoroéthane avec le mélange physique de référence. Le mode opératoire pour obtenir le modafinil entrant dans ces formulations est celui de la demande internationale WO 2010/112702. 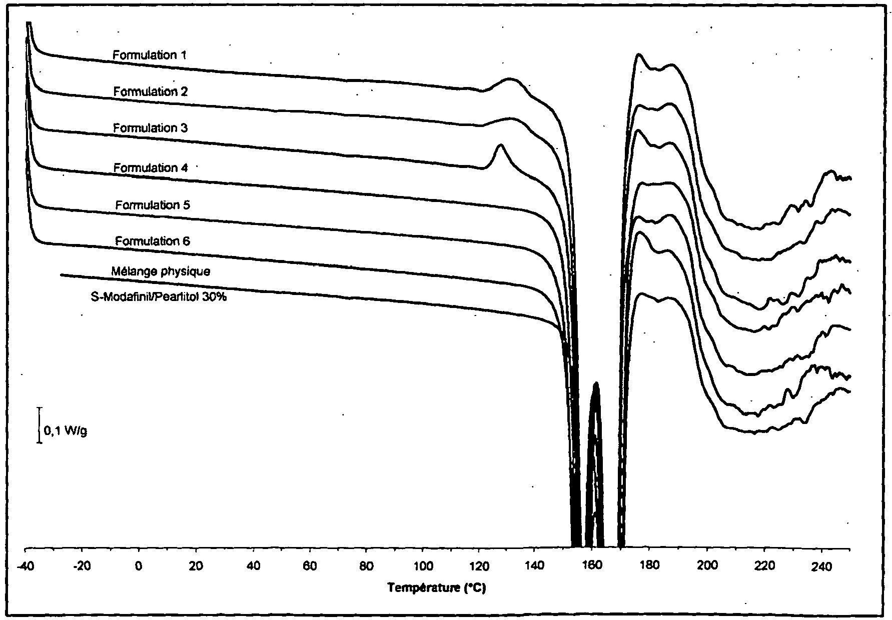 Différents clichés de particules de S modafinil ont été réalisés à l'aide d'un microscope à balayage électronique (MEB) à partir de formulations S-Modafinil/Mannitol à 30% ; ces clichés sont reproduits ci-après, l'échantillon 2 ayant été obtenu avec le C0₂ comme solvant supercritique et l'échantillon 4 avec le tétrafluoroéthane.

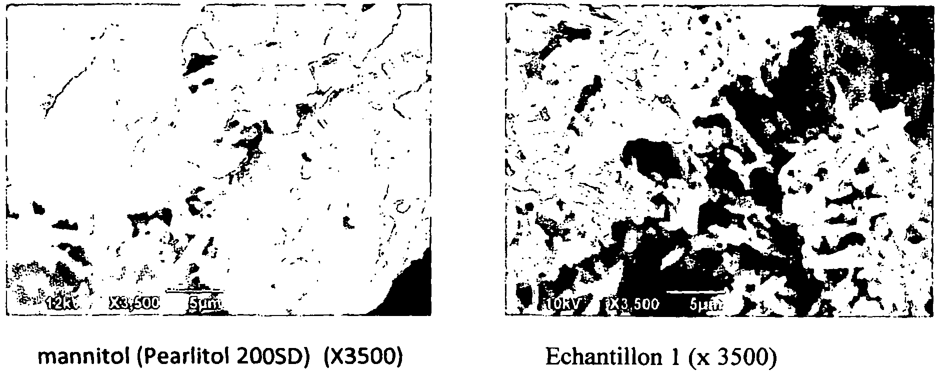

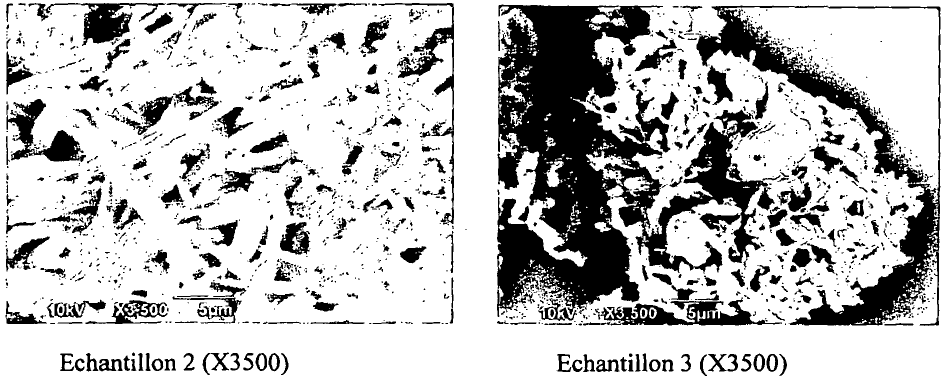

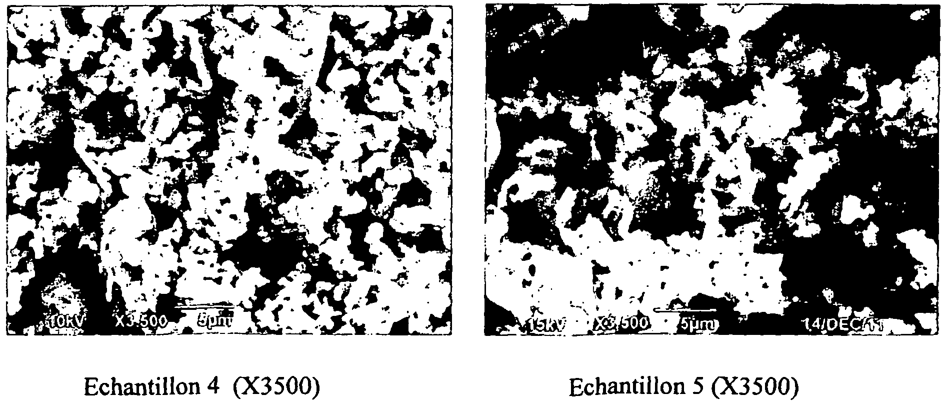

Les figures suivantes illustrent successivement la comparaison des cinétiques de dissolution des formulations à 30% de S modafinil / mannitol préparées par le procédé CO₂ supercritique (milieu HCl 0,1N) - échantillon 2 - et par le procédé utilisant le tétrafluoroéthane - échantillon 4.

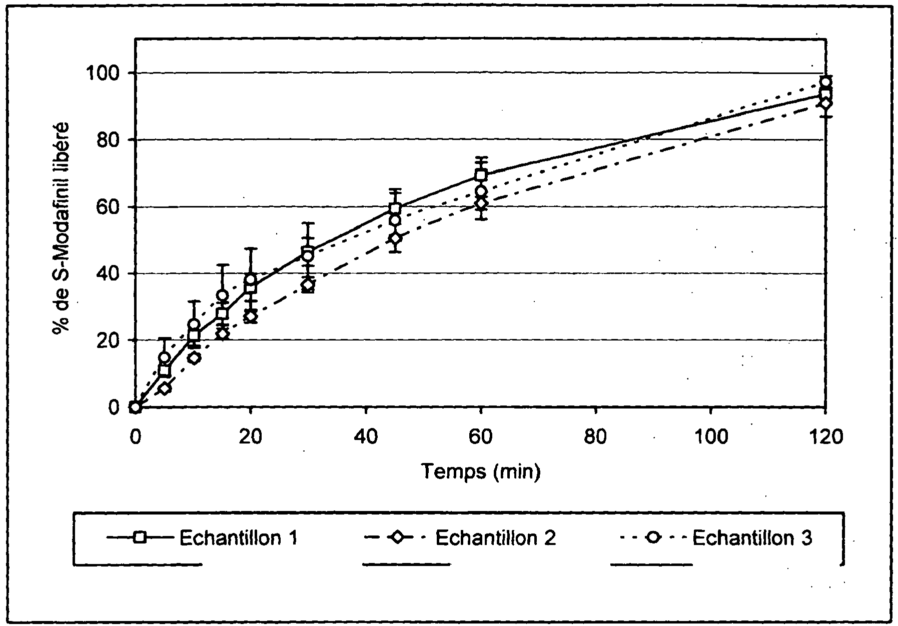

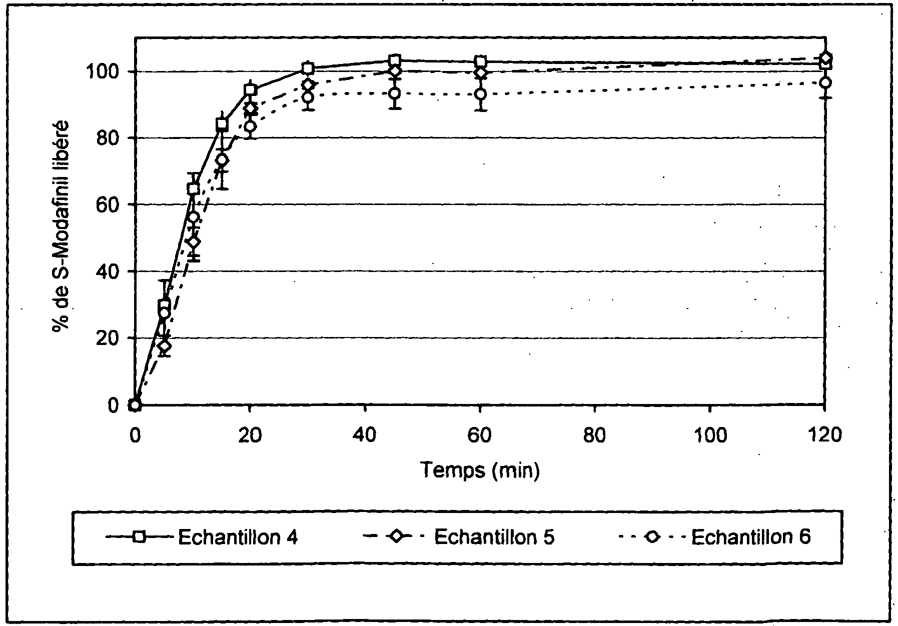

Les résultats analytiques des formulations confirment les résultats obtenus lors des études précédentes bien que l'excipient de capture ait été modifié :
- préparation de formulations uniformes en titre avec une bonne efficacité de capture des particules de S-modafinil ;
- les profils DSC sont similaires aux profils des formulations préparées dans les mêmes conditions opératoires lors des tâches précédentes ;
- les observations en MEB des formulations semblent indiquer que le S modafinil est cristallisé sous la forme de particules de taille de l'ordre du micromètre ;
- les cinétiques de dissolution sont reproductibles pour les deux types de formulations et similaires aux résultats obtenus précédemment. La vitesse de dissolution des formulations CO₂ supercritique est assez lente. Les formulations avec le tétrafluoroéthane présentent un profil de dissolution très rapide;

Les préparations obtenues précédemment ont été formulées de manière à obtenir des comprimés dosés à 2mg de S modafinil, ces comprimés étant destinés à être administrés chez le rat lors d'une étude de pharmacocinétique.

Le procédé utilisé est le suivant :
- après pesée de chacun des composants, les excipients sont introduits successivement par ordre croissant pondéral dans un mélangeur ;
- le mélange est ensuite tamisé pour éliminer les amas éventuels ;
- le mélange obtenu est ensuite compacté puis calibré sur une grille d'ouverture 1,25 mm à 250 tours/mn ;
- on procède ensuite à la compression sur machine à comprimer SVIAC.
Formule DASC00512 :

| **Matières Premières** | ***g*** | % |
|---|---|---|
| Echantillon 4 | 5,000 | 55,64 |
| Aérosil 200 | 1,250 | 13,91 |
| PVP XL | 0,358 | 3,99 |
| PVP K30 | 0,765 | 8,51 |
| Pearlitol 400 DC | 1,523 | 16,95 |
| St de mg | 0,090 | 1,00 |
| **TOTAL** | 8,987 | 100,00 |

Les résultats de dissolution apparaissent sur la figure suivante dans laquelle on compare le Modiodal, la formulation 4 (poudre), et l'échantillon DASC 00512 (comprimés avec dissolution en paniers).

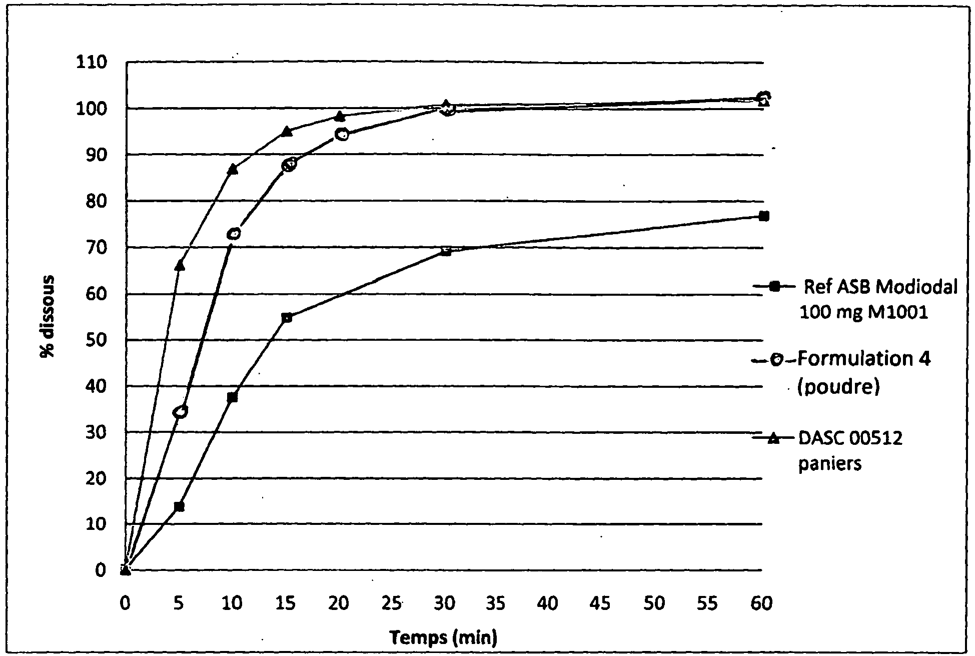

La formulation du S modafinil obtenue selon le procédé du fluide supercritique confirme bien qu'elle permet à la fois une libération très rapide (inférieure à 1 heure) et un effet à durée limitée (entre 3 et 4 heures) : c'est cette double caractéristique qui rend intéressante la formulation selon la présente invention.

Cette formulation consiste en des granules constituant un support, par exemple du mannitol ou du lactose anhydre, ou tout autre support inerte sur la surface desquelles ont été absorbées des particules de S modafinil obtenues à l'aide de la technologie de fluide supercritique.

Avantageusement, les comprimés obtenus à partir de cette formulation seront dosés entre 25 à 200 mg de S modafinil, et encore plus avantageusement entre 50 et 100 mg.
La caractéristique précitée permet d'utiliser ces comprimés dès lors que le consommateur a besoin d'une vigilance accrue à court terme, c'est-à-dire très rapidement (moins d'une heure) pendant une brève période (3 à 4 heures par exemple).

## Revendications

1. Procédé d'obtention d'une composition pharmaceutique **caractérisé en ce qu'**il consiste à solubiliser le S modafinil dans un fluide à l'état supercritique, puis après détente dudit fluide à récupérer ledit S modafinil en le faisant absorber en surface par un support inerte se présentant sous forme de granules.

2. Procédé selon la revendication 1 **caractérisé en ce que** le fluide supercritique est le CO₂.

3. Procédé selon la revendication 1 **caractérisé en ce que** le fluide supercritique est le tétrafluoroéthane.

4. Procédé selon l'une quelconque des revendications 1 à 3 **caractérisé en ce que** ledit support est du mannitol.

5. Procédé selon l'une quelconque des revendications 1 à 3 **caractérisé en ce que** ledit support est du lactose anhydre.

6. Composition pharmaceutique obtenue selon le procédé de l'une quelconque des revendications 1 à 5.

7. Composition pharmaceutique selon la revendication 6 **caractérisée en ce qu'**elle se présente sous forme orale.

8. Composition pharmaceutique selon la revendication 7 **caractérisée en ce que** ladite forme orale contient de 25 à 200 mg de S modafinil:

9. Composition pharmaceutique selon la revendication 8 **caractérisée en ce que** ladite forme orale contient de 50 à 100 mg de S modafinil.

10. Composition pharmaceutique selon l'une quelconque des revendications 7 à 9 **caractérisée en ce que** ladite forme orale est un comprimé.

## Patentansprüche

1. Verfahren zum Erlangen einer pharmazeutischen Zusammensetzung, **dadurch gekennzeichnet, dass** das Verfahren in dem Solubilisieren von S-Modafinil in einem Fluid im superkritischen Zustand und dann nach Entspannen des Fluids zum Zurückgewinnen des besagten S-Modafinils dem Absorbierenlassen dieses auf einer Oberfläche eines inerten Trägers, der die Form von Granulat aufweist, besteht.

2. Verfahren nach Anspruch 1, **dadurch gekennzeichnet, dass** das superkritische Fluid CO₂ ist.

3. Verfahren nach Anspruch 1, **dadurch gekennzeichnet, dass** das superkritische Fluid Tetrafluorethan ist.

4. Verfahren nach einem der Ansprüche 1 bis 3, **dadurch gekennzeichnet, dass** der besagte Träger Mannitol ist.

5. Verfahren nach einem der Ansprüche 1 bis 3, **dadurch gekennzeichnet, dass** der besagte Träger wasserfreie Laktose ist.

6. Pharmazeutische Zusammensetzung, die gemäß dem Verfahren nach einem der Ansprüche 1 bis 5 erlangt wurde.

7. Pharmazeutische Zusammensetzung nach Anspruch 6, **dadurch gekennzeichnet, dass** sie eine orale Form aufweist.

8. Pharmazeutische Zusammensetzung nach Anspruch 7, **dadurch gekennzeichnet, dass** die besagte orale Form 25 bis 200 mg S-Modafinil enthält.

9. Pharmazeutische Zusammensetzung nach Anspruch 8, **dadurch gekennzeichnet, dass** die besagte orale Form 50 bis 100 mg S-Modafinil enthält.

10. Pharmazeutische Zusammensetzung nach einem der Ansprüche 7 bis 9, **dadurch gekennzeichnet, dass** die besagte orale Form eine Tablette ist.

## Claims

1. Method for obtaining a pharmaceutical composition **characterised in that** it consists in dissolving the S modafinil in a fluid in the supercritical state, and then after expansion of the said fluid, recovering the said S modafinil by absorbing it at the surface by an inert carrier in the form of granules.

2. Method according to claim 1 **characterised in that** the supercritical fluid is C0₂.

3. Method according to claim 1 **characterised in that** the supercritical fluid is tetrafluoroethane.

4. Method according to any one of the claims 1 to 3 **characterised in that** the said carrier is mannitol.

5. Method according to any one of the claims 1 to 3 **characterised in that** the said carrier is anhydrous lactose.

6. Pharmaceutical composition obtainable by the method of any one of the claims 1 to 5.

7. Pharmaceutical composition according to claim 6 **characterised in that** it is in oral form.

8. Pharmaceutical composition according to claim 7 **characterised in that** the oral form contains 25 to 200 mg of S modafinil.

9. Pharmaceutical composition according to claim 8 **characterised in that** the said oral form contains 50 to 100 mg of S modafinil.

10. Pharmaceutical composition according to any one of the claims 7 to 9 **characterised in that** the oral form is a tablet.
